# EUROPEAN PATENT SPECIFICATION

(11) **EP 4 208 719 B1**
(45) Date of publication and mention of the grant of the patent: **30.10.2024**
(21) Application number: 21782815.1
(22) Date of filing: 02.09.2021
(51) Int. Cl.: G01N 33/543, G01N 33/574, G01N 33/58

(54) **SYSTEM, KIT, METHOD AND PROCESS FOR HANDLING A SAMPLE**
SYSTEM, KIT, VERFAHREN UND VERFAHREN ZUR HANDHABUNG EINER PROBE
SYSTEME, KIT, MÉTHODE ET PROCÉDÉ POUR MANIPULER UN ÉCHANTILLON

(30) Priority: 02.09.2020 IT 202000020890
(43) Date of publication of application: 12.07.2023
(73) Proprietor: Menarini Silicon Biosystems S.p.A., 40013 Castel Maggiore (BO) (IT)
(72) Inventor: MEDORO, Gianni, 40033 Casalecchio Di Reno (BO) (IT); CALANCA, Alex, 41037 Mirandola (MO) (IT)
(74) Representative: Studio Torta S.p.A.
(86) International application number: PCT/IB2021/058024
(87) International publication number: WO 2022/049520

(56) References cited:
- WO-A1-2014/152758
- US-A1- 2004 101 443
- US-A1- 2018 217 171
- MEDORO G. ET AL: "Use of the DEPArray platform to detect, isolate, and molecularly characterize pure tumor cells from peripheral blood samples enriched using the CellSearch system.", vol. 29, no. 15_suppl, 20 May 2011 (2011-05-20), US, pages 10616 - 10616, XP055798332, ISSN: 0732-183X, Retrieved from the Internet <URL:https://cdn2.hubspot.net/hub/304284/file-315243955-pdf/Doc/Medoro_2011_ASCO_poster.pdf?t=1452719851572> DOI: 10.1200/jco.2011.29.15_suppl.10616
- NEUMANN MARTIN HORST DIETER ET AL: "Isolation and characterization of circulating tumor cells using a novel workflow combining the CellSearch system and the CellCelector (TM)", vol. 33, no. 1, 17 May 2016 (2016-05-17), pages 125 - 132, XP055798336, ISSN: 8756-7938, Retrieved from the Internet <URL:https://api.wiley.com/onlinelibrary/tdm/v1/articles/10.1002%2Fbtpr.2294> DOI: 10.1002/btpr.2294

## Description

This patent application claims priority from Italian patent application no. 102020000020890 filed on September 2, 2020.

### TECHNICAL FIELD

The present invention concerns a system, a kit, a method and a process for handling a sample.

### BACKGROUND OF THE INVENTION

In the field of the handling of samples containing particles (in particular, cells) the need is felt to obtain samples enriched with one or more types of particles.

In this context, one of the methods currently used entails manually inserting in a test tube containing a sample of biological material (for example blood or blood from which at least part of the plasma has been removed) a specific reagent containing ferromagnetic particles functionalized with ligands specific for particular particles of the sample so that said particles are selectively marked.

Subsequently, a magnetic field is applied so that the marked particles are concentrated on the test tube wall. At this point, an operator inserts a pipette into the test tube and manually sucks out the part of the sample which is not close to the wall. In this way a sample enriched in the marked particles is obtained (inside the test tube), which can (subsequently) be separated from the ferromagnetic particles using appropriate reagents.

Particularly interesting examples of this method are described in the patent U6620627 and in the article Optimization of ferrofluids and protocols for enrichment of breast tumor cells in blood, Paul A. Liberti, Chandra G. Rao, Leon W. M. M. Terstappen, Journal of Magnetism and Magnetic Materials 225 (2001), 301-307.

It should be noted, however, that the method described above is complex, long, laborious and imprecise. In fact, the activities required are time-consuming: you have to be careful not to touch the test tube wall with the pipette, not to shake the test tube too much and not to suck the sample out too quickly.

Errors in one or more of these aspects increase the risk of sucking out also part (or all) of the marked particles.

Moreover, even the most highly trained operator cannot guarantee that the pipette (and in particular, its tip) will be kept at a sufficient distance from the wall and that the correct suction speed will be applied.

To remedy these drawbacks the use of completely automated systems has been proposed that reproduce the activities of the operator in a quicker and more reproducible manner.

However, these systems are very complex and costly.

The object of the present invention is to provide a system, a kit, a method and a process for handling a sample which overcome, at least partly, the drawbacks of the known art and at the same time are easy and inexpensive to produce.

### SUMMARY

According to the present invention, a system, a kit, a method and a process are provided for handling a sample as claimed in the following independent claims and, preferably, in any one of the claims depending directly or indirectly on the independent claims.

According to some non-limiting embodiments, the particles of the first type (mentioned in the claims) are chosen from the group consisting of: CTC, CEC (Circulating Endothelial Cells), CMC (Circulating Melanoma Cells), CMMC (Circulating Multiple Myeloma Cells), tdEV (tumor derived Extra Vescicles), exosomes, fetal cells, stem cells, other rare cells, viruses, bacteria, FFPE (Formalin-Fixed Paraffin-Embedded), spermatozoa, blood cells, epithelial cells, DNA, RNA and microspheres.

In particular, the particles of the first type (mentioned in the claims) are cells.

In some non-limiting cases, the particles of the first type are chosen from the group consisting of: CTC, CEC (Circulating Endothelial Cells), CMC (Circulating Melanoma Cells), CMMC (Circulating Multiple Myeloma Cells), tdEV (tumor derived Extra Vescicles), exosomes, fetal cells, stem cells, other rare cells, viruses, bacteria, FFPE (Formalin-Fixed Paraffin-Embedded), spermatozoa, blood cells, epithelial cells, DNA and RNA.

For example, the particles of the first type (mentioned in the claims) are chosen from the group consisting of: tumor cells, white blood cells (WBCs), stromal cells, spermatozoa, circulating tumor cells (CTCs), spores, fetal cells, liposomes, exosomes, epithelial cells, erythroblasts, trophoblasts, viruses, bacteria, erythrocytes (and a combination thereof). In some specific non-limiting cases, the particles of the first type are circulating tumor cells (CTC).

The cells can be live or fixed.

Unless explicitly specified to the contrary, in the present text the following terms have the meaning indicated below.

By ferromagnetic particle we mean a corpuscle having largest dimension less than approximately 10 µm (advantageously, less than approximately 1 µm - in particular, approximately 100 nm). According to some preferred but non-limiting embodiments, the largest dimension of the ferromagnetic particles is at least approximately 40 nm (in particular, up to approximately 200 nm).

The dimensions of the particles can be measured in standard mode with graduated scale microscopes or ordinary microscopes used with slides (on which the particles are deposited) with graduated scale.

In the present text, by dimensions of a particle we mean the length, width and thickness of the particle.

### BRIEF DESCRIPTION OF THE FIGURES

The invention is described below with reference to the attached drawings, which illustrate non-limiting embodiment examples thereof, in which:
- figure 1 is a schematic lateral view of a system in accordance with the present invention;
- figure 2 is a perspective view of the system of figure 1 with some parts removed for clarity;
- figure 3 is a front view of a detail of the system of figure 1;
- figure 4 is a section along the line IV-IV of the detail of figure 3;
- figure 5 is a front view of the detail of figure 3 with some parts removed for clarity;
- figure 6 is a section along the line VI-VI of the detail of figure 5;
- figure 7 is a front view of a detail of figure 3 with some parts removed for clarity;
- figure 8 is a section along the line VIII-VIII of the detail of figure 7;
- figure 9 is a plan view on an enlarged scale of a part of figures 3 and 7;
- figure 10 is a section along the line X-X of the part of figure 9;
- figure 11 schematically illustrates an example of possible use of the system of the preceding figures;
- figures 12 to 14 schematically illustrate tests which are performed to verify the operation of the system of the preceding figures; and
- figure 15 schematically illustrates what happens during a step of the method in accordance with the present invention.

### DETAILED DISCLOSURE

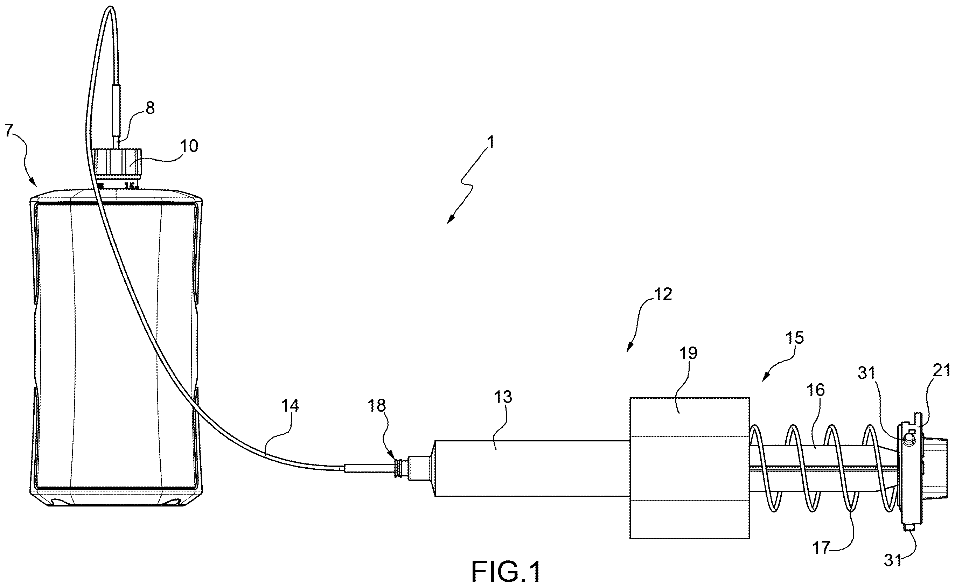

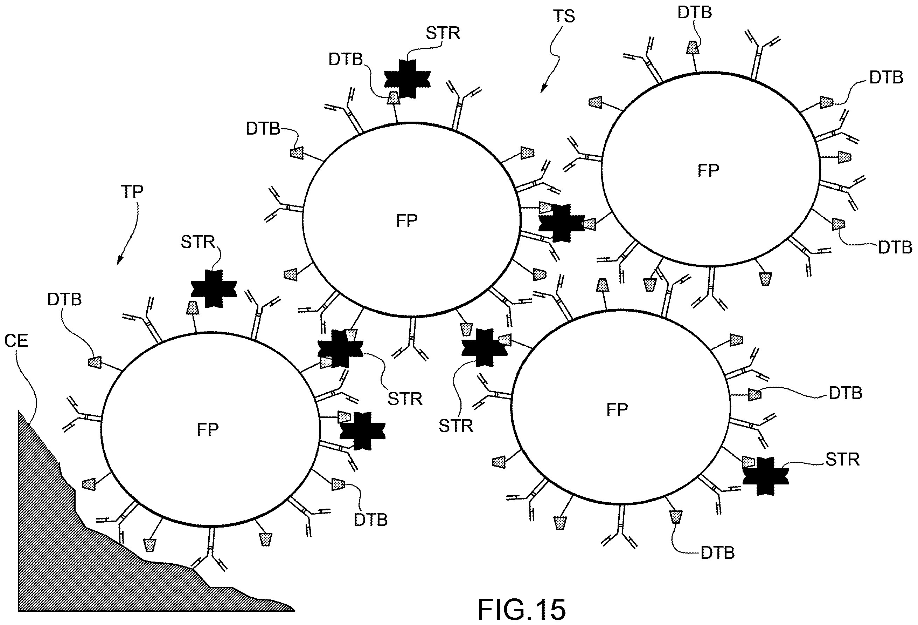

In figure 1, in accordance with a first aspect of the present invention, the number 1 indicates overall a system for handling a treated sample TS (in particular, of biological origin - see figure 15) comprising treated particles TP, each of which comprises a particle CE of a first type and at least a ferromagnetic particle FP bound to said particle CE of the first type.

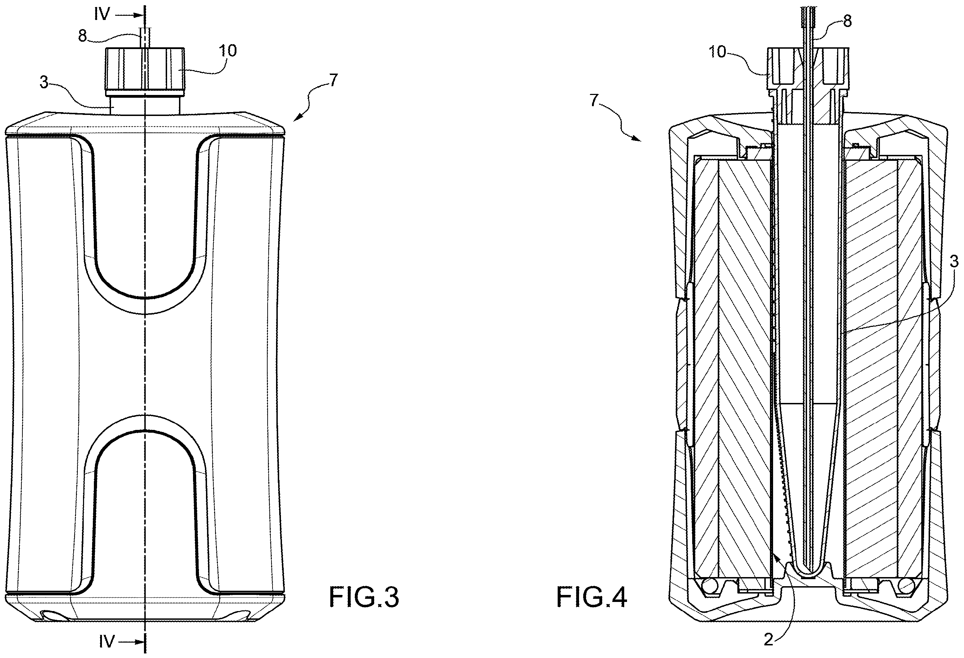

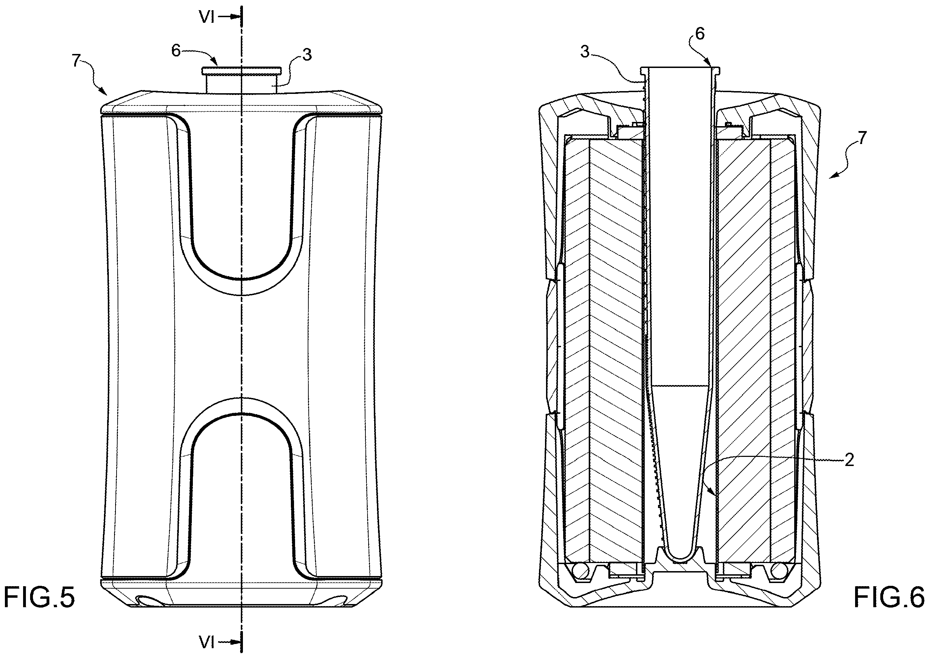

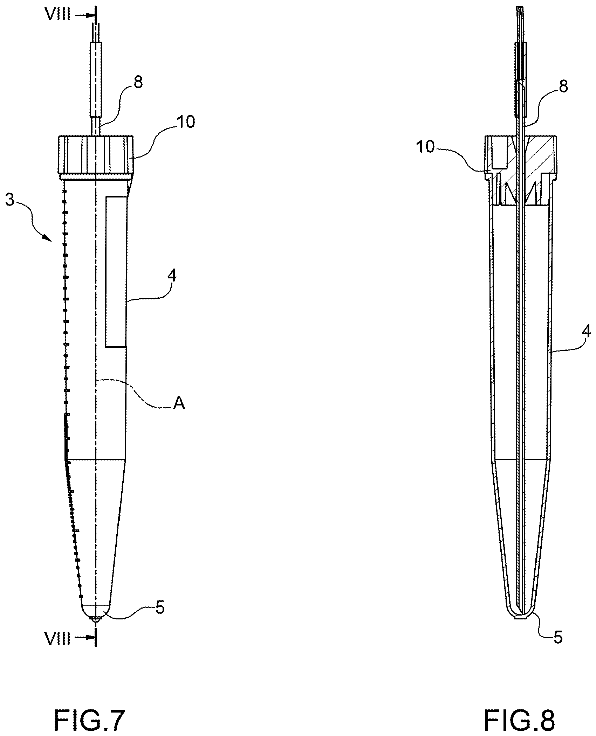

The system 1 comprises a treatment chamber 2 (see, in particular, figures 4 and 6); a container 3 (in particular, a test tube), which is configured to (designed to) contain the treated sample TS, and is arranged (at least partially) in the treatment chamber 2 and has at least one side wall 4 (see also figures 7 and 8), a bottom 5 (in particular, a bottom wall 5) and an (end) opening 6 opposite the bottom 5; and a magnetic device 7 configured to (designed to) create at least a magnetic field in at least a part of the treatment chamber 2 so as to move at least part of the treated particles TP onto the (in particular so as to come into contact and maintain contact with the) side wall 4 of the container 3.

More precisely but not necessarily, the treatment chamber 2 is part of the magnetic device 7 which, according to some non-limiting embodiments, comprises a magnetic quadrupole.

According to some non-limiting embodiments, the magnetic device 7 comprises at least one electromagnet (in particular, four electromagnets).

Alternatively (or additionally), the magnetic device 7 comprises at least one permanent magnet (in particular, four permanent magnets).

Advantageously but not necessarily, the magnetic device 7 (in particular, each electromagnet and/or permanent magnet) is configured to generate (on its surface - in particular in the axial symmetry point) a magnetic field of at least 3000 Gauss (in particular, at least 4000 Gauss; in particular, up to 7000 Gauss; more in particular, the magnetic field can be measured by means of a Hall effect magnetometer - for example by means of the instrument GM07 by Hirst Magnetic Instruments Ltd. (Tesla House, Tregoniggie, Falmouth, Cornwall).

According to some non-limiting embodiments, the container 3 (see, in particular, figures 6 to 8) has a first end, at which the opening 6 is arranged, and a second end, opposite the first end and at which the bottom 5 is arranged. In particular, the side wall 4 is tapered towards the second end (in particular, towards the bottom 5).

In some non-limiting cases, there is no interruption between the bottom 5 and the side wall 4.

According to some non-limiting embodiments, the container 3 has a substantially circular (inner) cross-section (in particular, the side wall 4 extends circularly about a longitudinal extension axis A of the container 3).

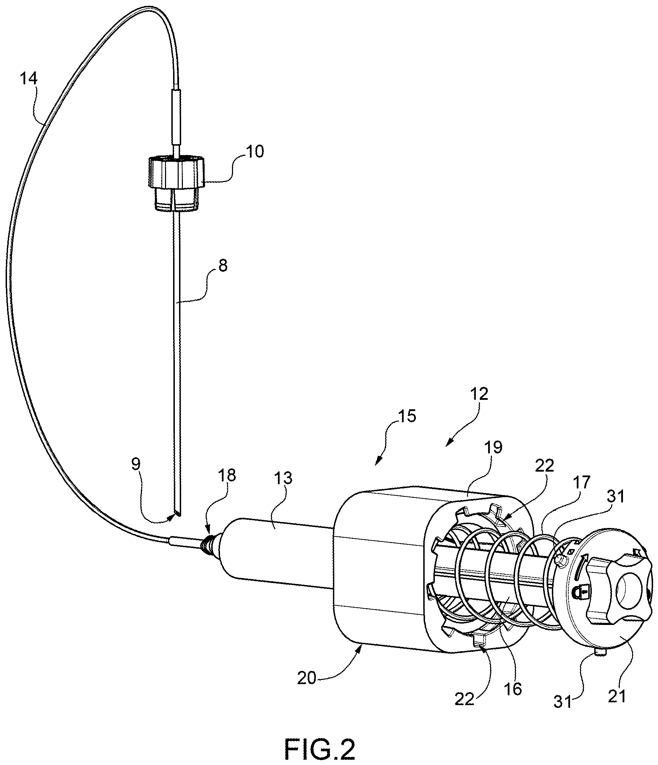

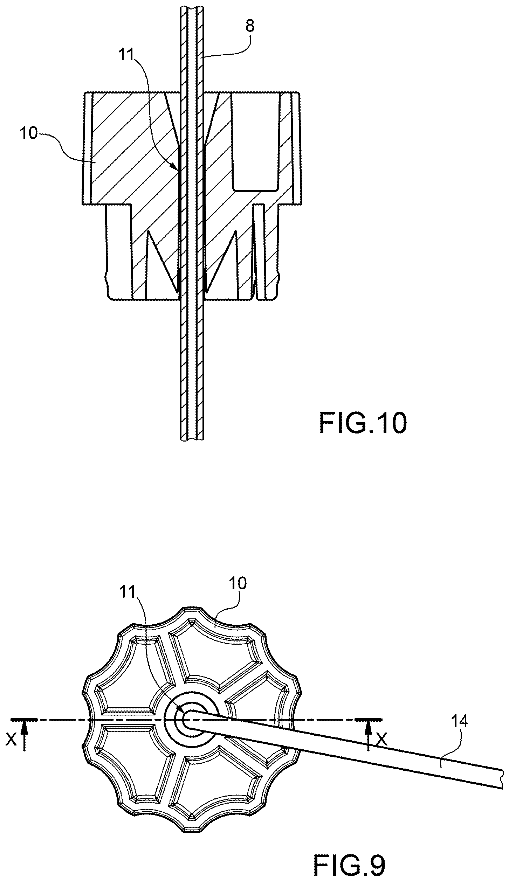

The system 1 further comprises a cannula 8, which has an open end 9 arranged inside the container 3; an alignment device 10, arranged in a substantially fixed manner in the area of the container 3 and having a through hole 11 (see, in particular, figure 9), through which the cannula 8 extends; and a suction device 12 (figures 1 and 2), which is configured to (designed to) create a depression inside the cannula 8 so that a part of the treated sample TS is carried through the cannula 8 out of the container 3 (and out of the treatment chamber 2).

In particular, the cannula 8 extends through the opening 6 of the container 3.

The cannula 8 and the alignment device 10 (in particular, the through hole 11) are configured so that (the cannula 8 extends - at least partially - inside the container 3 and) the open end 9 is arranged inside the container 3 separated (spaced) from the side wall 4 of the container 3.

It should be noted that, in this way, it has been experimentally observed that the possibilities of treated particles TP being sucked through the cannula 8 are reduced. More precisely, it has been observed that this result is obtained thanks to the structure of the alignment device 10 and of the cannula 8. In these cases, there is no need for complex robotic devices and the operator can insert the cannula 8 through the alignment device 10 (in particular, through the through hole 11) inside the treatment chamber 2 (more precisely, inside the container 3) at a good speed and with reduced risks of the cannula coming into contact with the side wall 4 and therefore causing movement of the treated particles TP which can, subsequently, be sucked through the cannula 8.

In other words, the alignment device 10 (in particular, the through hole 11) acts surprisingly as a guide for insertion of the cannula 8 into the treatment chamber 2, making the activities of the operator quicker and more precise.

According to some non-limiting embodiments, the cannula 8 and the alignment device 10 (in particular, the through hole 11) are configured so that the cannula 8 extends substantially parallel to the longitudinal extension axis A (in particular the axis A) of the container 3.

Advantageously but not necessarily, the open end 9 is in contact with the bottom 5. In this way, the vertical position of the cannula 8 is guaranteed by the support of the bottom part 5.

In particular (see for example figures 2, 4 and 8), the open end 9 extends diagonally relative to the longitudinal extension of the cannula 8 (more in particular, the end 9 extends diagonally relative to the axis A).

In this way, also when the end 9 is in contact with the bottom 5, fluid can pass through the end 9.

Advantageously but not necessarily, the cannula 8 extends through the through hole 11 in a removable manner. In other words, the cannula 8 can be inserted into or removed from the alignment device 10 (more precisely, the container 3) by causing it to go through the through hole 11.

In particular, the through hole 11 is configured so that, in use, while the cannula 8 is inserted through the through hole 11, the open end 9 of the cannula 8 (in particular - all - the cannula 8) is kept spaced from the side wall 4.

Advantageously but not necessarily, the through hole 9 has an inner surface which extends around the cannula 8 (in particular in contact with an outer surface of the cannula 8), in particular for at least approximately 0.5 cm of the longitudinal extension of the cannula 8 (more in particular, parallel to the axis A).

Additionally or alternatively, the inner surface of the through hole extends around the cannula 8 for at least two and a half times the width (more precisely, the diameter) of the cannula 8.

According to non-limiting embodiments, the through hole 11 has a width (internal diameter) greater than the width (external diameter) of the cannula 8 by less than approximately 1 mm (in particular, less than approximately 0.5 mm; more in particular, less than approximately 0.3 mm; even more in particular, at least approximately 0.1 mm).

In some non-limiting cases, the through hole 11 has a substantially circular (in particular, substantially constant) cross-section.

In particular, the cannula 8 is kept substantially in position thanks to the contact between the inner surface of the through hole 11 and the outer surface of the cannula 8. In particular, as better described above, the cannula 8 is kept substantially vertically in position, thanks to the contact with the bottom 5.

Advantageously but not necessarily, the cannula 8 is substantially rigid (in other words, it does not significantly deform during use - see the method described below - and/or when it is inserted through the through hole 11).

According to some preferred non-limiting embodiments, the cannula 8 is substantially straight. Additionally or alternatively, the through hole 11 is substantially straight.

In particular, the cannula 8 and the through hole 11 extend in the longitudinal extension direction of the container 3 (in particular, parallel to the axis A - more in particular, along the axis A).

Advantageously but not necessarily, the alignment device 10 is arranged at the opening 6 of the container 3. In this way, the alignment device 10 (in particular, the through hole 11 thereof) can be more easily accessed by the cannula 8 (and therefore insertion of the cannula 8 by the operator into the container 3 is facilitated).

In particular, the alignment device 10 partially closes (so as not to allow air through) the opening 6.

Advantageously but not necessarily, the alignment device 10 is arranged at the container 3 (in particular, at the opening 6) in a removable manner. In this way, the treated sample TS (or a sample to be treated) can be inserted into and removed from the container 3 more easily.

According to some non-limiting embodiments, the alignment device 10 (in particular, the through hole 11) and the cannula 8 are configured so that the cannula 8 is arranged separate (spaced) from the side wall 4.

Advantageously but not necessarily, the system 1 (in particular, the suction device 12) also comprises a collection chamber 13 (see, in particular, figures 1 and 2) which is connected to the cannula 8 in a fluid-carrying manner and arranged outside the treatment chamber 2 (and the container 3). The suction device 12 is configured to (designed to) create a depression inside the cannula 8 so that the above-mentioned part of the treated sample TS is carried through the cannula 8 to the collection chamber 13.

According to some non-limiting embodiments, the treated sample TS (of biological origin) comprises (in particular, is) any biological liquid.

Advantageously but not necessarily, the treated sample TS comprises (in particular, is) blood, plasma, saliva, urine, liquid biopsy, a swab containing cells (in particular, of tissue) in suspension (resuspended) and/or culture medium containing cells (in particular, of tissue) in suspension (resuspended).

According to some non-limiting embodiments, the treated sample TS comprises (in particular, is) blood, plasma, saliva, urine and a combination thereof.

In some specific non-limiting cases, the treated sample TS comprises (in particular, is) blood and/or plasma (in particular, blood).

According to some non-limiting embodiments, the system 1 (in particular, the suction device 12) also comprises a duct 14 that connects the cannula 8 to the collection chamber 13 in a fluid-carrying manner.

Advantageously but not necessarily, the suction device 12 and the cannula 8 (and, if necessary, the duct) are configured so as to obtain (when the open end 9 is immersed in water) a water flow speed through the open end 9 (through the cannula 8) up to (more precisely, less than) approximately 30 mL/min, in particular up to (more precisely, less than) approximately 15 mL/min, more in particular up to (more precisely, less than) approximately 13 mL/min.

In this way the risk of withdrawing treated particles TP from the container 3 is further reduced.

According to some non-limiting embodiments, the suction device 12 and the cannula 8 (and, in particular, said duct 14) are configured so as to obtain (when the open end is immersed in water) a water flow speed through the open end 9 (through the cannula 8) of at least approximately 1 mL/min, more in particular, at least approximately 3 mL/min, even more in particular, at least approximately 4 mL/min.

The flow speed is calculated as mean flow speed occurring in the suction of 10mL of water at ambient temperature (and external pressure of 1 atm).

Advantageously but not necessarily, the suction device 12 (and, in particular, the cannula 8; and, more in particular, the duct 14) is configured to (designed to) suck up to approximately 50 mL (in particular, up to approximately 30 mL; more in particular, up to approximately 15 mL) of liquid through the open end 9 (through the cannula 8).

Additionally or alternatively, the suction device 12 (and, in particular, the collection chamber 13) is configured to (designed to) suck at least approximately 20 µL (in particular, at least approximately 1 mL; more in particular, at least approximately 3mL) of liquid through the open end 9 (through the cannula 8).

According to some non-limiting embodiments, the suction device 12 comprises (in particular, is) a device chosen from the group consisting of: a peristaltic pump, a vacuum pump, a syringe pump, a syringe activated by preloaded spring or a syringe operated by the force of gravity.

In some cases (like the one illustrated in figures 1 and 2), the suction device 12 comprises (in particular, is) a syringe activated by preloaded spring.

In particular (in these cases - as better illustrated in figure 2), the suction device 12 comprises a syringe 15 which, in turn, is provided with the collection chamber 13 and a piston 16 (sliding inside the collection chamber 13), and a spring 17 configured to (designed to) impart a movement to the piston 16 and, consequently, create a depression inside the collection chamber 13. Said depression is transferred to the cannula 8 (in particular, through the duct 14) so as to suck the above-mentioned part of the treated sample TS from the container 3.

According to some specific but non-limiting embodiments, the spring 17 (is circular and) has an internal diameter of approximately 28-34 mm (in particular, approximately 31 mm), an external diameter of approximately 31-37 mm (in particular, approximately 34 mm), a wire with diameter of approximately 1.2-1.7 mm (in particular, approximately 1.5 mm) and a length of approximately 100-115 mm (in particular, approximately 107 mm).

In particular, the spring 17 is made of stainless steel.

In some cases, the spring 17 has an elastic constant of approximately 220 N/m to approximately 250 N/m (in particular, approximately 234 N/m).

In particular, the spring 17 is configured to (designed to) impart to the piston 16 a movement away from a first end 18 of the collection chamber 13.

In particular, the duct 14 extends from the first end 18.

Advantageously but not necessarily, the suction device 12 further comprises an abutment element 19, which is mounted integral with a flange of the collection chamber 13 arranged at a second end 20 of the collection chamber 13 opposite the first end 18, so as to define a support for an end of the spring 17. In particular, the suction device 12 also comprises a second abutment element 21 mounted integral with one end of the piston 16 external to the collection chamber 13 to define a support for the other end of the spring 17.

The abutment elements 19 and 21 are provided with grooves 22 and pins 31 that can be coupled to one another so as to maintain the abutment elements 19 and 21 linked to each other.

In particular, in use, an operator can push the piston 16 towards the first end 18 until the pins 31 enter the respective grooves 22. At this point, by rotating the abutment element 21, the coupling between the abutment elements 19 and 21 is stabilized and preloading of the spring 17 is obtained.

In this way, when appropriate, the operator, by rotating the abutment element 19, will activate the spring 17 which will move the piston 16 as described above.

According to some non-limiting embodiments, the container 3 has an inner volume of up to approximately 55 mL (in particular, up to approximately 30 mL; more in particular, up to approximately 17 mL; even more in particular, up to approximately 13 mL; in particular, of at least approximately 100 µL; more in particular, of at least approximately 1.5 mL; even more in particular, of at least approximately 8 mL).

According to some non-limiting embodiments, the cannula 8 has an internal diameter of up to approximately 12 mm (in particular, up to approximately 10 mm; in particular, up to approximately 1 mm; more in particular up to approximately 0.9 mm). In particular, the cannula 8 has an internal diameter of at least approximately 0.3 mm, more in particular, at least approximately 0.5 mm.

Additionally or alternatively, the duct 14 has an internal diameter up to approximately 12 mm (in particular, up to approximately 10 mm; in particular, up to approximately 1 mm; more in particular up to approximately 0.9 mm). In particular, the duct 14 has an internal diameter of at least approximately 0.3 mm, more in particular, at least 0.5 mm.

According to some non-limiting embodiments, the at least one (each) ferromagnetic particle FP (of a - each - treated particle TP) is bound to the (respective) particle CE of the first type by means of (at least) an antibody (specific for that particle). For example, if the particles CE of the first type are CTC, the antibody is bound (in particular, selectively) to the EPCAM (Epithelial Cell Adhesion Molecule).

With particular reference to figure 15, advantageously but not necessarily, in the treated particles TP several ferromagnetic particles FP are bound to one another so as to increase the response of said treated particles TP to the magnetic field. For example, for this purpose, the interaction between the desthiobiotin DTB and the Streptavidin STR can be exploited.

In accordance with a further aspect of the present invention, a kit is provided for handling a treated sample TS (in particular, as defined above).

In particular, the kit is configured to (designed to) provide the system 1 as described above.

The kit comprises: a magnetic device 7 having a treatment chamber 2 and configured to (designed to) create a magnetic field in the treatment chamber 2; a container 3 which is configured to (designed to) contain the treated sample TS and to be housed inside the treatment chamber 2, and has at least a side wall 4, a bottom 5 and an (end) opening 6 opposite the bottom 5; and a cannula 8, which has an open end 9.

The kit further comprises an alignment device 10, which is configured to (designed to) be mounted in a manner which is substantially fixed (but, in particular, removable - by an operator) at the container 3 and has a through hole 11 configured to (designed to) be engaged by the cannula 8 and keep the cannula 8 in a substantially fixed position and with an orientation such that the cannula 8 is spaced from the side wall 4; and a suction device 12 which is configured to (designed to) create a depression inside the cannula 8 so that a part of the treated sample TS is carried through the cannula 8 out of the container 3 and out of the treatment chamber 2.

According to some non-limiting embodiments, the kit comprises a duct 14 configured to (designed to) connect the cannula 8 and the suction device 12 in a fluid-carrying manner.

According to some non-limiting embodiments, the alignment device 10 is configured to (designed to) be arranged in the area of the opening 6 of the container 3.

In particular, the duct 14 has a first end configured to (designed to) couple with the cannula 8 and a second end configured to (designed to) couple with the suction device 12.

In particular, the duct 8 is pliable.

Advantageously but not necessarily, the kit comprises ferromagnetic particles FP designed to (configured to) bind (in particular, in a selective manner) with the particles CE of the first type.

In particular, the ferromagnetic particles are provided with at least one antibody configured to (designed to) bind (more in particular, in a selective manner) with particles CE of the first type. For example, if the particles CE of the first type are CTC, the antibody is designed to bind (in particular, selectively) with the EPCAM (Epithelial Cell Adhesion Molecule) .

According to some non-limiting embodiments, the kit further comprises a marker for marking (in particular, substantially selectively) some particles (in particular, the particles CE of the first type).

More precisely but not necessarily, the marker (in particular, is fluorescent and) emits at one or more given wavelengths (in particular, in the ultraviolet).

Advantageously but not necessarily, the marker is selected from the group consisting of: a marker for tumor cells (in particular, CTC), a marker for White Blood Cells (WBC), a marker for the nuclei of the cells and a combination thereof (namely, a mixture thereof).

According to some non-limiting examples, the marker is selected from the group consisting of: cytokeratin (in particular, for marking tumor cells - more in particular, for marking CTC), CD45 (PTPRC - protein tyrosine phosphatase, receptor type C), in particular for marking the white blood cells (WBC), DAPI (4',6-diamidino-2-phenylindole), in particular for marking the nuclei of the cells, and a combination thereof (namely a mixture thereof).

Advantageously but not necessarily, the various components of the above-mentioned kit are as defined above in relation to the system 1.

In accordance with a further aspect of the present invention, a handling method is provided for handling a treated sample TS.

The method comprises: a preliminary step, during which the treated sample TS (of biological origin) comprising treated particles TP, each comprising a particle CE of a first type and at least one ferromagnetic particle FP bound to the particle CE of the first type, is inside a container 3 having at least one side wall 4, a bottom 5 and an (end) opening 6 opposite the bottom 5; an insertion step, during which the container 3 is inserted in a treatment chamber 2 of a magnetic device 7; and a coupling step, which is subsequent to the preliminary step and during which an alignment device 10 having a through hole 11 is coupled stably to the container 3.

Advantageously but not necessarily, the insertion step is subsequent to the preliminary step.

Advantageously but not necessarily, the preliminary step, the insertion step and the coupling step are performed by an operator (in particular, by hand).

The method further comprises a positioning step, which is subsequent to the coupling step and during which a cannula 8 having an open end 9 is made to slide through the through hole 11 without touching the side wall 4 until reaching a given position (in particular, so as to come into contact with the bottom 5), in which the open end 9 is arranged inside the container 3 separated (spaced) from the side wall 4; an activation step, which is subsequent to the insertion step and during which the magnetic device 7 creates (in particular, is operated so as to create) a magnetic field which brings at least part of the treated particles TP into contact with the side wall 4; and a suction step, which is subsequent to the positioning step and to the operation step and during which a suction device 12 creates a depression inside the cannula 8 so that a part of the treated sample TS is carried through the cannula 8 out of the container 3 (and out of said treatment chamber 2) leaving (at least a portion of the) at least part of the treated particles TP inside the container 3 (in particular, in contact with the side wall 4).

Advantageously but not necessarily, the positioning step (the operation step) and the suction step are performed by an operator (in particular, by hand). In particular, during the suction step, the operator activates the suction device 12.

Advantageously but not necessarily, the method is implemented by means of the kit described above.

According to some non-limiting embodiments, the treated sample TS comprises particles of a second type (not illustrated), at least a major part of which is carried through the cannula 8 out of the container 3, during the suction step.

In particular, the ferromagnetic particles FP are selectively bound to the particles CE of the first type and not to the particles of the second type.

In some non-limiting cases, the particles CE of the first type are those of interest (in particular, they are further handled and/or analyzed). In these cases, in particular, the particles of the second type are simply discarded.

Alternatively, the particles of the second type are those of interest (in particular, they are further handled and/or analyzed). In these cases, in particular, the particles CE of the first type are simply discarded.

According to some non-limiting embodiments the at least one (each) ferromagnetic particle FP (of one - each - treated particle TP), is bound to the (respective) particle CE of the first type by means of (at least) one antibody (specific for that particle).

According to some non-limiting embodiments, the method also comprises a decoupling step, during which at least part of the ferromagnetic particles FP are detached from the particles CE of the first type. In these cases, for example, if the interaction between the desthiobiotin DTB and the Streptavidin STR has been exploited, the biotin can be used.

Advantageously but not necessarily, the treated sample TS comprises particles of a further type different from the particles CE of the first type. In these cases, the method, in particular, comprises a step of marking, during which a marker binds in a substantially selective manner (in particular, relative to the particles of the further type) with the particles CE of the first type or in a substantially selective manner (in particular, relative to the particles CE of the first type) with the particles of the further type. The marker improves the distinction between the particles CE of the first type and the particles of the further type.

For example, the marker comprises fluorescent molecules with one or more given wavelengths (in particular, in the ultraviolet).

In particular, the marker comprises antibodies designed to selectively bind with the particles CE of the first type or with the particles of the further type. More in particular, the marker comprises (is) molecules consisting (each) of (at least) an antibody (designed to selectively bind with the particles CE of the first type or with the particles of the further type) and (at least) a fluorophore.

Advantageously but not necessarily, the marker is selected from the group consisting of: a marker for tumor cells (in particular, CTC), a marker for white blood cells (WBC), a marker for the nuclei of the cells and a combination thereof (namely a mixture thereof).

According to some non-limiting examples, the marker is selected from the group consisting of: cytokeratin (in particular, for marking tumor cells), CD45 (PTPRC - protein tyrosine phosphatase, receptor type C), in particular for marking the white blood cells (WBC), DAPI (4',6-diamidino-2-phenylindole), in particular for marking cells with nuclei and a combination thereof.

Advantageously but not necessarily, the marking step is subsequent to the suction step.

In particular, during the marking step, a solution containing the marker is placed in the container 3 (inside which the treated particles TP are arranged - more precisely, the treated sample TS is arranged).

Advantageously but not necessarily, while the solution containing the marker is placed in the container 3, the treated particles TP are kept in contact with the side wall 4 thanks to the magnetic field generated by the magnetic device 7.

According to some non-limiting embodiments, during the marking step, the above-mentioned marker binds in a substantially selective manner (in particular, relative to the particles of the further type) with the particles CE of the first type and a further marker (different from the above-mentioned marker) binds in a substantially selective manner (in particular, relative to the particles CE of the first type) with the particles of the further type.

In these cases, in particular, the further marker comprises antibodies designed to selectively bind with the particles CE of the first type. More in particular, the further marker comprises (is) molecules consisting (each) of (at least) an antibody (designed to selectively bind with the particles CE of the first type) and (at least) a fluorophore. Moreover, the further marker comprises antibodies designed to selectively bind with the particles of the further type. More in particular, the further marker comprises (is) molecules consisting of (at least) an antibody (designed to selectively bind with the particles of the further type) and (at least) a further fluorophore.

According to some non-limiting examples, the further marker is selected from the group consisting of: cytokeratin (in particular, for marking tumor cells), CD45 (PTPRC - protein tyrosine phosphatase, receptor type C), in particular for marking the white blood cells (WBC), DAPI (4',6-diamidino-2-phenylindole), in particular for marking cells with nuclei, and a combination thereof.

According to some non-limiting embodiments, the marking step comprises a sub-step of permeabilization, during which the permeability of external (cell) membranes of the particles CE of the first type (or of the further type) is increased so as to allow the marker (or the further marker) to enter the inside of the particles CE of the first type (or of the further type).

In accordance with some non-limiting variations, the increase in permeability of the membranes is obtained by using an appropriate reagent.

Advantageously but not necessarily, the method also comprises a fixing step subsequent or previous to (or simultaneous with) the marking step (in particular, previous to the activation step). Typically but not necessarily, the fixing step is subsequent to the preliminary step.

In accordance with some non-limiting variations, the fixing is obtained by using an appropriate reagent.

Advantageously but not necessarily, the method also comprises a removal step, which is (at least partially) subsequent to the marking step and during which at least part of the solution containing the marker is removed from the container 3.

In particular, during the removal step, the treated particles TP are kept in contact with the side wall 4 thanks to the magnetic field generated by the magnetic device 7.

According to some non-limiting embodiments, the method also comprises a washing step, which is (at least partially) subsequent to the removal step and during which a buffer is inserted in the container 3 and, subsequently, at least partially removed.

In particular, during the washing step, the treated particles TP are kept in contact with the side wall 4 thanks to the magnetic field generated by the magnetic device 7.

According to some non-limiting variations, the particles of the further type are part of the particles of the second type (for example, left inside the container 3 after the suction step). Alternatively, the particles of the further type are different from the particles of the second type.

In accordance with a further aspect of the present invention, a process is provided for handling an initial sample (of biological origin) comprising particles CE of a first type (e.g., CTC). The process comprises a treatment step, during which ferromagnetic particles FP able to selectively bind with the particles CE of the first type are combined with the particles CE of the first type so as to obtain a treated sample TS comprising treated particles TP, each of which comprises a particle CE of a first type and at least a ferromagnetic particle FP bound to the particle CE of the first type; and a handling method as described above.

According to some non-limiting embodiments, the ferromagnetic particles FP are provided with at least one antibody configured to (designed to) bind (in particular, in a selective manner) with the particles CE of the first type. In particular, during the treatment step the ferromagnetic particles FP bind with the respective particles CE of the first type by means of the cited antibody.

Advantageously but not necessarily, the treatment step precedes the preliminary step. In particular, during the treatment step the ferromagnetic particles FP are inserted inside the container 3, in which the particles CE are arranged (in particular, the initial sample is arranged).

According to some non-limiting embodiments, the initial sample (of biological origin) comprises (in particular, is) any biological liquid.

Advantageously but not necessarily, the initial sample comprises (in particular, is) blood, plasma, saliva, urine, liquid biopsy, a buffer containing cells (in particular, of tissue) in suspension (resuspended) and/or culture medium containing cells (in particular, of tissue) in suspension (resuspended).

According to some non-limiting embodiments, the initial sample comprises (in particular, is) blood, plasma, saliva, urine and a combination thereof.

In some specific non-limiting cases, the initial sample comprises (in particular, is) blood and/or plasma (in particular, blood).

Advantageously but not necessarily, the process also comprises a removal step, which is prior to the treatment step (and, in particular, prior to the preliminary step) and during which at least a major portion of the (of plasma contained in the) initial sample is removed and separated from the particles CE of the first type (from the container 3). In particular, 0.5 to 1 mL of plasma remains inside the container 3.

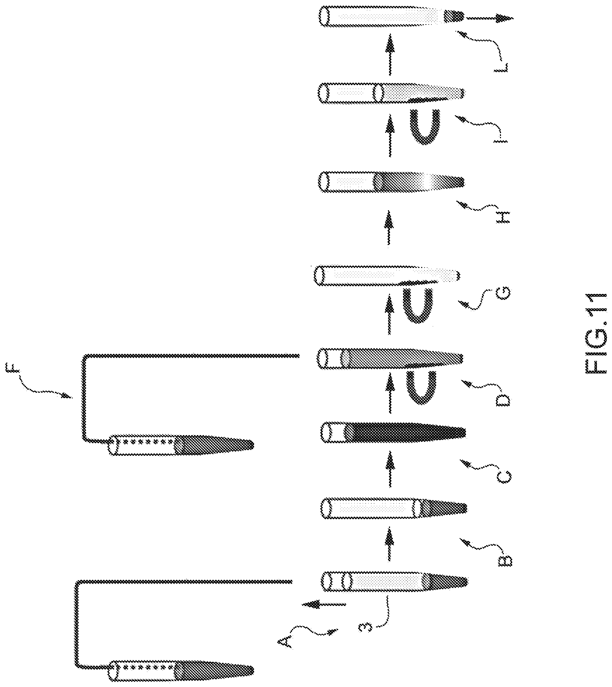

Figure 11 schematically illustrates a non-limiting example of the above-mentioned process, in which the initial sample (blood) with the addition of a buffer is centrifuged (to separate the plasma) (A); the diluted plasma is removed (B); ferromagnetic particles FP (and further buffers) are added (C) so as to obtain the treated sample TS containing the treated particles TP; a magnetic field (D) is applied so as to carry the treated particles onto the side wall 4 of the container 3; a portion of the treated sample TS (containing non-marked cells) is removed (F); the treated particles TP are washed (G); dye reagents are added to the treated particles TP (H), which are washed again after a new application of the magnetic field (I); the treated particles TP are resuspended in a small volume (L) and loaded in an analysis and/or counting device.

In accordance with the above, the subject of the present invention allows for the enrichment (and marking) of particles (in particular, cells) of interest in a simple, rapid and inexpensive manner, and the removal of negative particles (in particular, cells) (it is possible to use both the positive sample fraction - which includes the particles of interest - and the negative fraction - with the negative particles).

The result obtained can be used to prepare the material for an analysis/sorting system/device (for example prior to the DEPArray^{™} device produced by the same applicant) or for purposes of cell culture, genetic analysis, counting, cytometry etc.

Further characteristics of the present invention will become clear from the following description of merely illustrative and non-limiting examples.

### Example 1

### This example describes the results of some experimental tests carried out

The subject of the present invention was tested and comparative tests were carried out with the results obtained with a fully automated system currently on the market (the system called CellSearch^{™}).

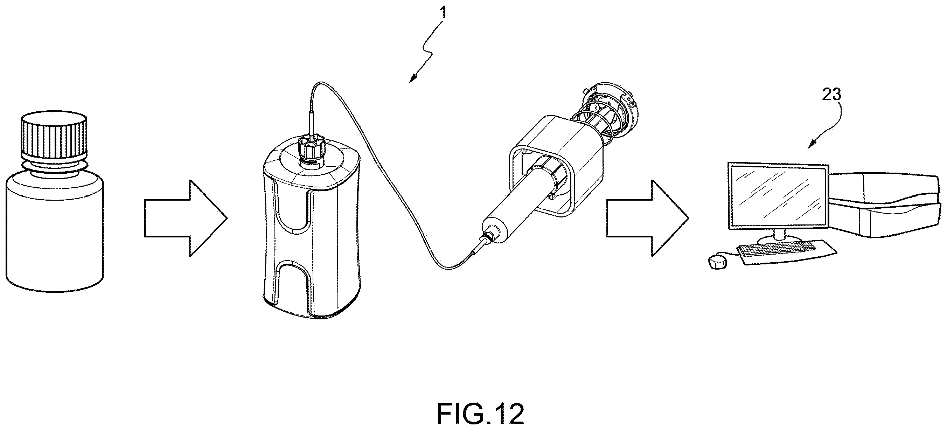

With particular reference to figure 12, tests were carried out starting from a sample of epithelial cells suspended in a buffer solution. The results are shown in table 1 below.

**Table 1**

| | CM | CS1 | CS2 |
|---|---|---|---|
| Number of tests | 11 | 2 | 12 |
| Average type of samples marked with first color upon entry | 1088 | 1088 | 1108 |
| Average type of samples marked with second color upon entry | 50 | 50 | 57 |
| Average type of samples marked with first color upon exit | 899 (82.6%) | 985 (90.5%) | 968 (87.4%) |
| Average type of samples marked with second color upon exit | 45.8 (91.6%) | 45 (90%) | 54.8 (96.2%) |
| S.D. of average of samples marked with first color upon exit | 16.60% | 1.90% | 2.60% |
| S.D. of average of samples marked with second color upon exit | 29% | 14.10% | 14.80% |

It should be noted that in the above table 1 CM indicates the results obtained with a system 1 in accordance with the present invention; CS1 and CS2 indicate the results obtained with two different versions of the completely automated system currently on the market (CellSearch^{™}); S.D. indicates the standard deviation.

In figure 12, the reference number 23 indicates a cell counting machine (in particular, the Celltracks Analyzer II^{®}).

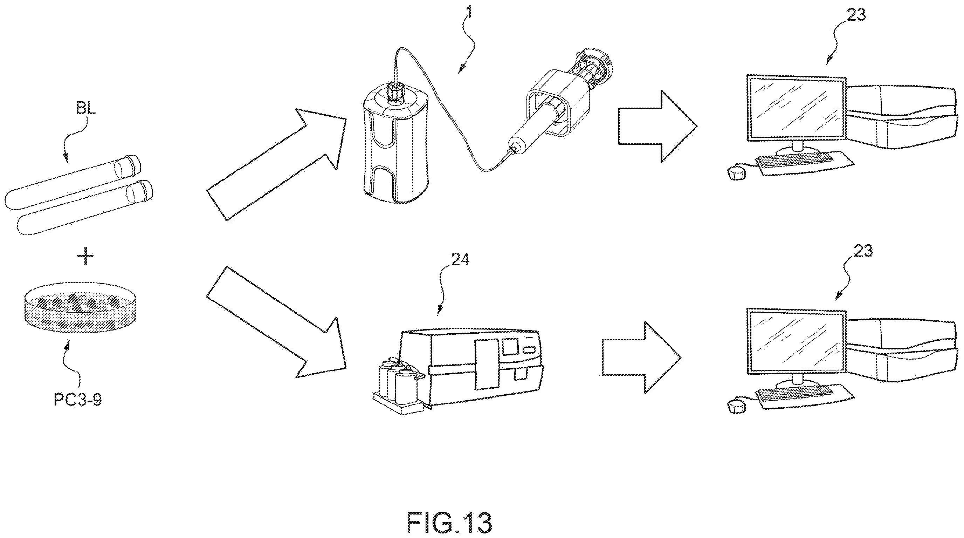

With particular reference to figure 13, tests were performed starting from a sample obtained from blood (BL) taken from a healthy donor, to which tumor cells (PC3-9) were added. The results are shown below in table 2.

**Table 2**

| | CM | CS1 |
|---|---|---|
| Number of tests | 8 | 8 |
| Number of PC3-9 upon entry | 1087 | 1087 |
| PC3-9 averages upon exit | 45.85% | 42.04% |
| S.D. PC3-9 averages upon exit | 7.44% | 3.41% |

Note that in the above table 2, CM indicates the results obtained with a system 1 in accordance with the present invention.

In figure 13, the reference number 23 indicates a cell counting machine (in particular, the Celltracks Analyzer II^{®}) and the reference number 24 indicates the above-mentioned completely automated system currently on the market (CELLTRACKS^{®} AUTOPREP^{®} System).

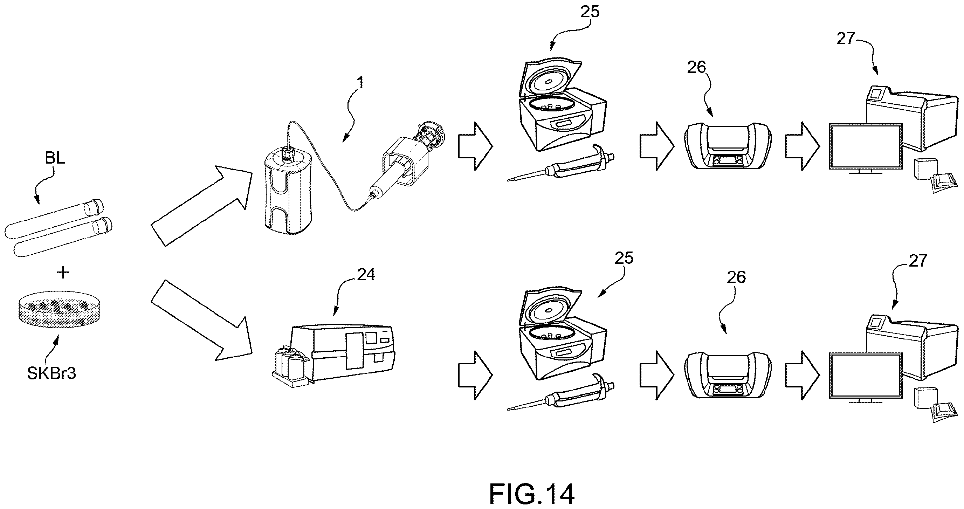

With particular reference to figure 14, tests were performed starting from a sample obtained from blood (BL) taken from a healthy donor, to which tumor cells (SKBr3) were added. The results are shown below in table 3.

**Table 3**

| | CM | CS1 |
|---|---|---|
| Number of tests | 15 | 15 |
| Number of samples upon entry | 13 tests with 542, 2 tests with 2168 | 13 tests with 542, 2 tests with 2168 |
| Average samples upon exit | 51.67% | 61.25% |
| S.D. average samples upon exit | 10.69% | 15.67% |

Note that in the above table 3, CM indicates the results obtained with a system 1 in accordance with the present invention; CS1 indicates the results obtained with a completely automated system currently on the market (CellSearch^{™}) ; S.D. indicates the standard deviation.

The events indicated in tables 1 and 3 above indicate single SKBr3 or clusters of SKBr3 (which tend to agglomerate).

In figures 13 and 14, the reference number 23 indicates a cell counting machine (in particular, the Celltracks Analyzer II^{®}); the reference number 24 indicates the above-mentioned completely automated system currently on the market; the reference number 25 indicates a washing system (in particular, a centrifuge for 15 ml test tube and 1.5 ml PCR test tube: any supplier) ; the reference number 26 indicates a system for reduction of the volume (in particular, a VRNxT^{™} by Menarini Silicon Biosystems was used); the number 27 indicates a sorting device known under the trade name DEPArray^{™}.

As can be easily noted from the above experimental data, the subject of the present invention, despite its decidedly lower cost and complexity, had a comparable precision and, in some cases, was higher than that of the automated system currently on the market.

In the tests performed, the following were used:
anti-EpCAM ferrofluid containing a 0.022% suspension of magnetic particles bound to a mouse monoclonal antibody specific for the cell surface marker EpCAM present on the epithelial cells in a buffer containing 0.03% of bovine serum albumin (BSA) and conserving ProClin 300 at 0.05%.

### Dilution buffer containing buffer with 0.1% sodium azide.

The following procedure (indicating a non-limiting example of the method) was followed. 7.5 ml of blood were transferred to a test tube, diluted with 6.5 ml of buffer and, subsequently, stirred and centrifuged for approximately 10 minutes (at approximately 800 g). The plasma (supernatant) was removed by means of a pipette leaving approximately 0.5-1 ml and approximately 6 ml of dilution buffer were added.

After brief stirring of the test tube, 150 µl of reagent were added to improve the capture (0.02% reagent for controlled ferrofluidic aggregation, 0.5% BSA, 0.1% of sodium azide in buffer) and the above-mentioned ferrofluid (150 µl).

After brief stirring, the test tube was placed in the treatment chamber 2 of the magnetic device 7 (figures 1-10) for 10 minutes.

After further stirring (with removal of the test tube from the chamber), and a fixed residence in the treatment chamber 2 of approximately 20 minutes, the suction device 12 was mounted and operated (maintaining the test tube inside the magnetic device 7) to suck the negative fraction.

The sample obtained was then washed with dilution buffer using the magnetic device 7 following a similar method.

In order to allow recognition of the target cells by means of fluorescence images, the sample was further treated with permeabilization reagent, dye reagent (containing mouse monoclonal antibodies specific for cytokeratins bound to phycoerythrin (PE) and mouse monoclonal antibody anti-CD45 bound to allophycocyanin (APC)) and dye for nucleic acid (containing 4 '6-diamidino-2-phenylindole dihydrochloride (DAPI)), resuspending the sample to allow mixing of the reagents and incubating at ambient temperature for 20 minutes in the dark.

The sample was then washed by diluting with 2 ml of dilution buffer, keeping the test tube in the magnetic device 7 for approximately 15 minutes prior to the suction performed by means of the suction device 12. Lastly, the sample was fixed by means of a cell fixative and resuspended in the final volume.

The DEPArray^{™} and the CellSearch^{™} were used following the respective manufacturer instructions.

The samples processed with the DEPArray^{™} were washed with a further buffer before resuspension in the final volume.

## Claims

1. A system for handling a treated sample (TS) comprising treated particles (TP), each comprising a particle (CE) of a first type and at least one ferromagnetic particle (FP) bound to said particle (CE) of the first type;
the system (1) comprises a treatment chamber (2); a container (3), which is configured to contain said treated sample (TS), is at least partially placed in said treatment chamber (2) and has at least a side wall (4), a bottom (5) and an opening (6) opposite the bottom (5); a magnetic device (7), which is configured to create at least one magnetic field in at least part of the treatment chamber (2) so as to move at least part of the treated particles (TP) onto the side wall (4) of said container (3); a cannula (8), which has an open end (9) placed inside said container (3); an alignment device (10), which is placed in a substantially fixed manner in the area of said container (3) and has a through hole (11), through which said cannula (8) extends; and a suction device (12), which is configured to create a depression inside said cannula (8) so that part of said treated sample (TS) is carried, through said cannula (8), out of said container (3);
said cannula (8) and said alignment device (10), in particular, said through hole, being configured so that said open end (9) is placed inside said container (3) spaced from said side wall (4) of the container (3).

2. The system according to claim 1, wherein said alignment device (10) is placed in the area of said opening (6) of said container (3); the alignment device (10), in particular, said through hole, and said cannula (8) being configured so that the cannula (8) is spaced from the side wall (4).

3. The system according to claim 1 or 2, wherein said alignment device (10) is placed in the area of said container (3) in particular, in the area of said opening (6) of the container, in a removable manner; said cannula (8) extends through said through hole (11) in a removable manner; said through hole (11) being configured so that, in use, while the cannula (8) is inserted through the through hole (11), said open end (9) of the cannula (8) is kept at a distance from said side wall (4); in particular, said cannula (8) being substantially rigid.

4. The system according to any one of the preceding claims, and comprising a collection chamber (13), which is connected to said cannula (8) in a fluid-carrying manner and is placed on the outside of said treatment chamber (2); the suction device (12) is configured to create a depression inside said cannula (8) so that said part of said treated sample (TS) is carried, through said cannula (8), to the collection chamber (13); in particular, the system (1) also comprises a duct (14), which connects said cannula (8) to said collection chamber (13) in a fluid-carrying manner.

5. The system according to any one of the preceding claims, wherein said suction device (12) and said cannula (8) and in particular, said duct, are configured so as to obtain a water flow speed through said open end (9) up to about 30 mL/min, in particular, up to about 15 mL/min; in particular, the suction device (12) and the cannula (8) and in particular said duct, are configured so as to obtain a water flow speed through the open end (9) of at least about 1 mL/min, more in particular, of at least about 4 mL/min.

6. The system according to any one of the preceding claims, wherein the suction device (12) and in particular, the collection chamber (13); more in particular, the cannula (8); and, more in particular, said duct, is configured so as to suck up to about 50 mL, in particular, up to about 30 mL, of liquid through said open end (9); in particular, said treatment chamber (2) and said container (3) each have an inner volume up to about 55 mL, in particular, up to about 13 mL.

7. The system according to any one of the preceding claims, wherein said through hole (11) has an inner surface, which extends around said cannula (8) along at least about 0.5 cm of the longitudinal extension of the cannula (8).

8. The system according to any one of the preceding claims, wherein said cannula (8) is substantially straight; said through hole (11) is substantially straight.

9. A kit for handling a treated sample (TS); the kit is configured to obtain a system (1) according to any one of the preceding claims; the kit comprises:
a magnetic device (7) having a treatment chamber (2) and configured to create a magnetic field in the treatment chamber (2) ;
a container (3), which is configured to contain said treated sample (TS) and to be housed inside said treatment chamber (2) and has at least a side wall (4), a bottom (5) and an opening (6) opposite the bottom (5);
a cannula (8), which has an open end (9);
an alignment device (10), which is configured to be mounted in a substantially fixed manner in the area of said container (3) and has a through hole (11) which is configured to be engaged by said cannula (8) and to keep the cannula (8) in a substantially fixed position and with a substantially fixed orientation and such that the cannula (8) is spaced apart from said side wall (4) of the container (3); and
a suction device (12), which is configured to create a depression inside said cannula (8) so that part of said treated sample (TS) is carried, through said cannula (8), out of said container (3).

10. The kit according to claim 9 and comprising a duct (14), which is configured to connect said cannula (8) and said suction device (12) in a fluid-carrying manner; said alignment device (10) being configured to be placed in the area of said opening (6) of the container (3); in particular, the duct (14) has a first end, which is configured to be coupled to the cannula (8), and a second end, which is configured to be coupled to the suction device (12); in particular, the duct (14) is pliable.

11. The kit according to claim 9 or 10, wherein said cannula (8) has an inner diameter up to about 12 mm, in particular up to about 1 cm; in particular, the cannula (8) has an inner diameter of at least about 0.3 mm, more in particular, at least about 0.5 mm.

12. The kit according to any one of the claims from 9 to 11 and comprising ferromagnetic particles (FP), which are provided with at least one antibody configured to be bound to particles (CE) of the first type; the kit further comprises at least one marker chosen from the group consisting of: a marker for tumor cells, in particular CTCs, a marker for white blood cells (WBCs), a marker for the nuclei of the cells, and a combination thereof; in particular, the marker is selected from the group consisting of: cytokeratin, CD45 (PTPRC - protein tyrosine phosphatase, receptor type C), DAPI (4',6-diamidino-2-phenylindole) and a combination thereof.

13. A method of handling a treated sample (TS); the method comprises:
a preliminary step, during which the treated sample (TS), which comprises treated particles (TP), each comprising a particle (CE) of a first type and at least one ferromagnetic particle (FP) bound to said particle (CE) of the first type, is in a container (3) having at least a side wall (4), a bottom (5) and an opening (6) opposite the bottom (5);
an insertion step, during which said container (3) is inserted into a treatment chamber (2) of a magnetic device (7);
a coupling step, which is subsequent to the preliminary step and during which an alignment device (10) having a through hole (11) is coupled to said container (3) in a stable manner;
a positioning step, which is subsequent to the coupling step and during which a cannula (8) having an open end (9) is caused to go through the through hole (11) without touching said side wall (4) until it reaches a given position, in which said open end (9) is placed inside said container (3) separate from said side wall (4) of the container (3);
an activation step, which is subsequent to the insertion step and during which the magnetic device (7) creates a magnetic field, which causes at least part of said treated particles (TP) to come into contact with said side wall (4); and
a suction step, which is subsequent to the positioning step and to the activation step and during which a suction device (12) creates a depression inside said cannula (8) so that part of the treated sample (TS) is carried, through said cannula (8), out of said container (3) leaving at least a portion of said at least part of the treated particles (TP) inside the container.

14. The method according to claim 13 and implemented by means of the kit according to any one of the claims from 9 to 12; in particular, the treated sample (TS) comprises particles of a second type, at least a major part thereof being carried, through said cannula (8), out of said container (3) during said suction step; in particular, the particles (CE) of the first type are circulating tumor cells.

15. The method according to claim 13 or 14, wherein the particles (CE) of the first type are chosen from the group consisting of: CTCs, CECs (Circulating Endothelial Cells), CMCs (Circulating Melanoma Cells), CMMCs (Circulating Multiple Myeloma Cells), tdEVs (tumor derived Extra Vescicles), exosomes, fetal cells, stem cells, other rare cells, viruses, bacteria, FFPE (Formalin-Fixed Paraffin-Embedded), spermatozoa, blood cells, epithelial cells, DNA, RNA, microspheres.

16. The method according to any one of the claims from 13 to 15, wherein the treated sample (TS) comprises particles of a further type different from the particles (CE) of the first type; the method comprises a marking step, during which a marker is bound in a selective manner, in particular, relative to the particles of the further type, to the particles (CE) of the first type or in a selective manner, in particular, relative to the particles (CE) of the first type, to the particles of the further type; for example, the marker comprises fluorescent molecules with one or more given wavelengths.

17. A process of handling an initial sample comprising particles (CE) of a first type; the process comprises a treatment step, during which ferromagnetic particles (FP) capable of being selectively bound to said particles (CE) of the first type are mixed with the particles (CE) of the first type so as to obtain a treated sample (TS) comprising treated particles (TP), each comprising a particle (CE) of a first type and at least one ferromagnetic particle (FP) bound to said particle (CE) of the first type; and a method according to any one of the claims from 13 to 16.

18. The process according to claim 17, wherein the initial sample comprises a biological liquid selected from the group consisting of: blood, plasma, saliva, urine, liquid biopsy, a swab containing cells in suspension, a culture medium containing cells in suspension, and a combination thereof; in particular, the process also comprises a removal step, which is prior to the treatment step and during which at least a major part of the initial sample is removed and separated from the particles (CE) of the first type.

## Patentansprüche

1. System für eine Handhabung einer behandelten Probe (TS), umfassend behandelte Partikel (TP), jeweils umfassend ein Partikel (CE) eines ersten Typs und mindestens ein ferromagnetisches Partikel (FP), das an das Partikel (CE) des ersten Typs gebunden ist;
wobei das System (1) eine Behandlungskammer (2) umfasst, wobei ein Behälter (3), der konfiguriert ist, um die behandelte Probe (TS) zu enthalten, mindestens teilweise in der Behandlungskammer (2) platziert ist und mindestens eine Seitenwand (4), einen Boden (5) und eine Öffnung (6), die dem Boden (5) gegenüberliegt, aufweist, eine Magnetvorrichtung (7), die konfiguriert ist, um mindestens ein Magnetfeld in mindestens einem Teil der Behandlungskammer (2) zu erzeugen, um mindestens einen Teil der behandelten Partikel (TP) auf die Seitenwand (4) des Behälters (3) zu bewegen; eine Kanüle (8), die ein offenes Ende (9) aufweist, die im Inneren des Behälters (3) platziert ist; eine Ausrichtungsvorrichtung (10), die in dem Bereich des Behälters (3) im Wesentlichen auf eine fixierte Weise platziert ist und eine Durchgangsöffnung (11) aufweist, durch die sich die Kanüle (8) erstreckt; und eine Saugvorrichtung (12), die konfiguriert ist, um einen Unterdruck im Inneren der Kanüle (8) zu erzeugen, sodass ein Teil der behandelten Probe (TS) durch die Kanüle (8) aus dem Behälter (3) befördert wird;
wobei die Kanüle (8) und die Ausrichtungsvorrichtung (10), insbesondere das Durchgangsloch, konfiguriert sind, sodass das offene Ende (9) im Inneren des Behälters (3), beabstandet von der Seitenwand (4) des Behälters (3) platziert ist.

2. System nach Anspruch 1, wobei die Ausrichtungsvorrichtung (10) in dem Bereich der Öffnung (6) des Behälters (3) platziert ist; wobei die Ausrichtungsvorrichtung (10), insbesondere das Durchgangsloch und die Kanüle (8) konfiguriert sind, sodass die Kanüle (8) von der Seitenwand (4) beabstandet ist.

3. System nach Anspruch 1 oder 2, wobei die Ausrichtungsvorrichtung (10) in dem Bereich des Behälters (3), insbesondere in dem Bereich der Behälteröffnung (6), auf eine entfernbaren Weise platziert ist; sich die Kanüle (8) durch das Durchgangsloch (11) in einer entfernbaren Weise erstreckt; wobei das Durchgangsloch (11) konfiguriert ist, sodass im Gebrauch, während die Kanüle (8) durch das Durchgangsloch (11) eingeführt wird, das offene Ende (9) der Kanüle (8) in einem Abstand von der Seitenwand (4) gehalten wird; wobei insbesondere die Kanüle (8) im Wesentlichen starr ist.

4. System nach einem der vorstehenden Ansprüche, und umfassend eine Sammelkammer (13), die mit der Kanüle (8) auf eine fluidbefördernde Weise verbunden ist und auf der Außenseite der Behandlungskammer (2) platziert ist; wobei die Saugvorrichtung (12) konfiguriert ist, um einen Unterdruck im Inneren der Kanüle (8) zu erzeugen, sodass ein Teil der behandelten Probe (TS) durch die Kanüle (8) in die Sammelkammer (13) befördert wird; wobei insbesondere das System (1) ebenso eine Leitung (14) umfasst, welche die Kanüle (8) mit der Sammelkammer (13) auf eine fluidbefördernde Weise verbindet.

5. System nach einem der vorstehenden Ansprüche, wobei die Saugvorrichtung (12) und die Kanüle (8) und insbesondere der Kanal konfiguriert sind, sodass eine Wasserflussgeschwindigkeit durch das offene Ende (9) von bis zu etwa 30 ml/min, insbesondere bis zu etwa 15 ml/min, erhalten wird; wobei insbesondere die Saugvorrichtung (12) und die Kanüle (8) und insbesondere der Kanal konfiguriert sind, sodass eine Wasserdurchflussgeschwindigkeit durch das offene Ende (9) von mindestens etwa 1 ml/min, weiter insbesondere von mindestens etwa 4 ml/min erhalten wird.

6. System nach einem der vorstehenden Ansprüche,
wobei die Saugvorrichtung (12) und insbesondere die Sammelkammer (13); weiter insbesondere die Kanüle (8); und weiter insbesondere der Kanal konfiguriert sind, um bis zu etwa 50 ml, insbesondere bis zu etwa 30 ml Fluid durch das offene Ende (9) aufzusaugen; wobei insbesondere die Behandlungskammer (2) und der Behälter (3) jeweils ein Innenvolumen von bis zu etwa 55 ml, insbesondere von bis zu etwa 13 ml aufweisen.

7. System nach einem der vorstehenden Ansprüche, wobei das Durchgangsloch (11) eine Innenoberfläche aufweist, die sich um die Kanüle (8) entlang von mindestens etwa 0,5 cm der Längserstreckung der Kanüle (8) erstreckt.

8. System nach einem der vorstehenden Ansprüche, wobei die Kanüle (8) im Wesentlichen gerade ist; wobei das Durchgangsloch (11) im Wesentlichen gerade ist.

9. Kit für die Handhabung einer behandelten Probe (TS); wobei das Kit konfiguriert ist, um ein System (1) nach einem der vorstehenden Ansprüche zu erhalten, wobei das Kit umfasst:
eine Magnetvorrichtung (7), die eine Behandlungskammer (2) aufweist und konfiguriert ist, um ein Magnetfeld in der Behandlungskammer (2) zu erzeugen;
einen Behälter (3), der konfiguriert ist, um die behandelte Probe (TS) zu enthalten, und um im Inneren in der Behandlungskammer (2) untergebracht zu werden, und mindestens eine Seitenwand (4), einen Boden (5) und eine Öffnung (6), die dem Boden (5) gegenüberliegt, aufweist,
eine Kanüle (8), die ein offenes Ende (9) aufweist,
eine Ausrichtungsvorrichtung (10), die konfiguriert ist, um in dem Bereich des Behälters (3) im Wesentlichen auf eine fixierte Weise montiert zu werden, und die ein Durchgangsloch (11) aufweist, das konfiguriert ist, um durch die Kanüle (8) in Eingriff genommen zu werden, und um die Kanüle (8) in einer im Wesentlichen fixierten Position und mit einer im Wesentlichen fixierten Ausrichtung zu halten, derart, dass die Kanüle (8) von der Seitenwand (4) des Behälters (3) beabstandet ist, und
eine Saugvorrichtung (12), die konfiguriert ist, um im Inneren der Kanüle (8) einen Unterdruck zu erzeugen, sodass ein Teil der behandelten Probe (TS) durch die Kanüle (8) aus dem Behälter (3) befördert wird.

10. Kit nach Anspruch 9, umfassend einen Kanal (14), der konfiguriert ist, um die Kanüle (8) und die Saugvorrichtung (12) auf eine fluidbefördernde Weise zu verbinden; wobei die Ausrichtungsvorrichtung (10) konfiguriert ist, um in dem Bereich der Öffnung (6) des Behälters (3) platziert zu werden; wobei insbesondere der Kanal (14) ein erstes Ende aufweist, das konfiguriert ist, um mit der Kanüle (8) und einem zweiten Ende gekoppelt zu werden, das konfiguriert ist, um mit der Saugvorrichtung (12) gekoppelt zu werden; wobei insbesondere der Kanal (14) biegsam ist.

11. Kit nach Anspruch 9 oder 10, wobei die Kanüle (8) einen Innendurchmesser von bis zu etwa 12 mm, insbesondere von bis zu etwa 1 cm, aufweist, wobei insbesondere die Kanüle (8) einen Innendurchmesser von mindestens etwa 0,3 mm, weiter insbesondere von mindestens etwa 0,5 mm aufweist.

12. Kit nach einem der Ansprüche 9 bis 11, und umfassend ferromagnetische Partikel (FP), die mit mindestens einem Antikörper bereitgestellt sind, der konfiguriert ist, um an Partikel (CE) des ersten Typs gebunden zu werden;
wobei das Kit ferner mindestens einen Marker umfasst, gewählt aus der Gruppe bestehend aus: einem Marker für Tumorzellen, insbesondere CTCs, einem Marker für weiße Blutkörperchen (WBCs), einem Marker für die Kerne der Zellen und einer Kombination davon; wobei insbesondere der Marker ausgewählt ist aus der Gruppe bestehend aus: Zytokeratin, CD45 (PTPRC-Protein-Tyrosinphosphatase, Rezeptor Typ C), DAPI (4',6-Diamidino-2-phenylindol) und einer Kombination davon.

13. Verfahren für die Handhabung einer behandelten Probe (TS); wobei das Verfahren umfasst:
einen vorläufigen Schritt, während dessen die behandelte Probe (TS), die behandelte Partikel (TP) umfasst, jeweils umfassend ein Partikel (CE) eines ersten Typs und mindestens ein ferromagnetisches Partikel (FP), das an das Partikel (CE) des ersten Typs gebunden ist, in einem Behälter (3) ist, der mindestens eine Seitenwand (4), einen Boden (5) und eine Öffnung (6), die dem Boden (5) gegenüberliegt, aufweist,
einen Einführungsschritt, während dessen der Behälter (3) in eine Behandlungskammer (2) einer Magnetvorrichtung (7) eingeführt wird;
einen Kopplungsschritt, der auf den vorläufigen Schritt folgt und während dessen eine Ausrichtungsvorrichtung (10), die ein Durchgangsloch (11) aufweist, mit dem Behälter (3) auf eine stabile Weise gekoppelt wird;
einen Positionierungsschritt, der auf den Kopplungsschritt folgt und während dessen bewirkt wird, dass eine Kanüle (8), die ein offenes Ende (9) aufweist, durch das Durchgangsloch (11) geht, ohne ein Berühren der Seitenwand (4), bis sie eine vorgegebe Position erreicht, in der das offene Ende (9) im Inneren des Behälters (3) getrennt von der Seitenwand (4) des Behälters (3) platziert wird;
einen Aktivierungsschritt, der auf den Einführungsschritt folgt und während dessen die Magnetvorrichtung (7) ein Magnetfeld erzeugt, das bewirkt, dass mindestens ein Teil der behandelten Partikel (TP) mit der Seitenwand (4) in Kontakt kommt; und
einen Saugschritt, der auf den Positionierungsschritt und den Aktivierungsschritt folgt, und während dessen eine Saugvorrichtung (12) einen Unterdruck im Inneren der Kanüle (8) erzeugt, sodass ein Teil der behandelten Probe (TS) durch die Kanüle (8) aus dem Behälter (3) befördert wird, wobei mindestens ein Anteil des mindestens einen Teils der behandelten Partikel (TP) im Behälter verbleibt.

14. Verfahren nach Anspruch 13, und implementiert mittels des Kits nach einem der Ansprüche 9 bis 12; wobei insbesondere die behandelte Probe (TS) Partikel eines zweiten Typs aufweist, wobei mindestens ein Großteil davon durch die Kanüle (8) aus dem Behälter (3) während des Saugschritts befördert wird; wobei insbesondere die Partikeln (CE) des ersten Typs zirkulierende Tumorzellen sind.

15. Verfahren nach Anspruch 13 oder 14, wobei die Partikel (CE) des ersten Typs aus der Gruppe gewählt werden bestehend aus: CTCs, CECs (zirkulierende Endothelzellen), CMCs (zirkulierende Melanomzellen), CMMCs (zirkulierende multiple Myelomzellen), tdEVs (aus Tumoren stammende Extravesikel), Exosomen, fetalen Zellen, Stammzellen, anderen seltenen Zellen, Viren, Bakterien, FFPE (Formalin-fixiert, in Paraffin eingebettet), Spermien, Blutzellen, Epithelzellen, DNA, RNA, Mikrokügelchen.

16. Verfahren nach einem der Ansprüche 13 bis 15, wobei die behandelte Probe (TS) Partikel eines weiteren Typs umfasst, der sich von den Partikeln (CE) des ersten Typs unterscheidet; wobei das Verfahren einen Markierungsschritt umfasst, während dessen ein Marker, insbesondere bezogen auf die Partikel des weiteren Typs an die Partikel (CE) des ersten Typs, auf eine selektive Weise gebunden wird, oder insbesondere bezogen auf die Partikel (CE) des ersten Typs, an die Partikel des weiteren Typs auf eine selektive Weise gebunden wird; wobei zum Beispiel der Marker fluoreszierende Moleküle mit einer oder mehreren vorgegebenen Wellenlängen umfasst.

17. Vorgang zum Handhaben einer Ausgangsprobe, umfassend Partikel (CE) eines ersten Typs, wobei der Vorgang einen Behandlungsschritt umfasst, während dessen ferromagnetische Partikel (FP), die in der Lage sind an die Partikel (CE) des ersten Typs selektiv gebunden zu werden, mit den Partikeln (CE) des ersten Typs gemischt werden, um eine behandelte Probe (TS) zu erhalten, umfassend behandelte Partikel (TP), jeweils umfassend ein Partikel (CE) eines ersten Typs und mindestens ein ferromagnetisches Partikel (FP), das an das Partikel (CE) des ersten Typs gebunden ist; und ein Verfahren nach einem der Ansprüche 13 bis 16.

18. Vorgang nach Anspruch 17, wobei die Ausgangsprobe eine biologische Flüssigkeit umfasst, ausgewählt aus der Gruppe bestehend aus: Blut, Plasma, Speichel, Urin, Flüssigbiopsie, einem Abstrich, der Zellen in Suspension enthält, einem Kulturmedium, das Zellen in Suspension enthält, und einer Kombination davon; wobei insbesondere der Vorgang ebenso einen Entfernungsschritt umfasst, der vor dem Behandlungsschritt liegt und während dessen mindestens ein Hauptteil der Ausgangsprobe entfernt und von den Partikeln (CE) des ersten Typs getrennt wird.

## Revendications

1. Système de manipulation d'un échantillon traité (TS) comprenant des particules traitées (TP), chacun comprenant une particule (CE) d'un premier type et au moins une particule ferromagnétique (FP) liée à ladite particule (CE) du premier type ;
le système (1) comprend une chambre de traitement (2) ; un récipient (3), qui est conçu pour contenir ledit échantillon traité (TS), est au moins partiellement placé dans ladite chambre de traitement (2) et a au moins une paroi latérale (4), un fond (5) et une ouverture (6) à l'opposé du fond (5) ; un dispositif magnétique (7), qui est configuré pour créer au moins un champ magnétique dans au moins une partie de la chambre de traitement (2) de manière à déplacer au moins une partie des particules traitées (TP) sur la paroi latérale (4) dudit récipient (3) ; une canule (8), dont l'extrémité ouverte (9) est placée à l'intérieur dudit récipient (3) ; un dispositif d'alignement (10), qui est placé de manière sensiblement fixe dans la zone dudit récipient (3) et a un trou de traversant (11), à travers lequel s'étend ladite canule (8) ; et un dispositif d'aspiration (12), qui est conçu pour créer une dépression à l'intérieur de ladite canule (8) afin qu'une partie dudit échantillon traité (TS) soit transportée, à travers ladite canule (8), hors dudit récipient (3) ;
ladite canule (8) et ledit dispositif d'alignement (10), en particulier ledit trou traversant, sont conçus de manière à placer ladite extrémité ouverte (9) à l'intérieur dudit récipient (3) à distance de ladite paroi latérale (4) du récipient (3).

2. Système selon la revendication 1, dans lequel ledit dispositif d'alignement (10) est placé dans la zone de ladite ouverture (6) dudit récipient (3) ; le dispositif d'alignement (10),en particulier, ledit trou traversant, et ladite canule (8) étant conçus de manière à espacer la canule (8) de la paroi latérale (4).

3. Système selon la revendication 1 ou 2, dans lequel ledit dispositif d'alignement (10) est placé dans la zone dudit récipient (3), en particulier dans la zone de ladite ouverture (6) du récipient, de manière amovible ; ladite canule (8) s'étend à travers ledit trou traversant (11) de manière amovible ; ledit trou traversant (11) est conçu de manière à maintenir, en cours d'utilisation, lorsque la canule (8) est insérée à travers le trou traversant (11), ladite extrémité ouverte (9) de la canule (8) à distance de ladite paroi latérale (4) ; en particulier, ladite canule (8) est sensiblement rigide.

4. Système selon l'une quelconque des revendications précédentes, et comprenant une chambre de collecte (13), qui est reliée à ladite canule (8) de manière à transporter le fluide et qui est placée à l'extérieur de ladite chambre de traitement (2) ; le dispositif d'aspiration (12) est conçu pour créer une dépression à l'intérieur de ladite canule (8) afin que ladite partie dudit échantillon traité (TS) soit transportée, à travers ladite canule (8), vers la chambre de collecte (13) ; en particulier, le système (1) comprend également un conduit (14) qui relie ladite canule (8) à ladite chambre de collecte (13) en transportant le fluide.

5. Système selon l'une quelconque des revendications précédentes, dans lequel ledit dispositif d'aspiration (12) et ladite canule (8), et en particulier ledit conduit, sont conçus de manière à obtenir une vitesse d'écoulement de l'eau à travers ladite extrémité ouverte (9) jusqu'à 30 mL/min environ, en particulier jusqu'à 15 mL/min environ ; en particulier, le dispositif d'aspiration (12) et la canule (8), et en particulier ledit conduit, sont conçus de manière à obtenir une vitesse d'écoulement de l'eau à travers l'extrémité ouverte (9) d'au moins 1 mL/min environ, plus particulièrement d'au moins 4 mL/min environ.

6. Système selon l'une des revendications précédentes,
dans lequel le dispositif d'aspiration (12) et, en particulier, la chambre de collecte (13) ; plus particulièrement, la canule (8) ; et, plus particulièrement, ledit conduit, est conçu de manière à aspirer jusqu'à 50 ml environ, en particulier jusqu'à 30 mL environ, de liquide à travers ladite extrémité ouverte (9) ; en particulier, ladite chambre de traitement (2) et ledit récipient (3) ont chacun un volume interne allant jusqu'à 55 mL environ, en particulier jusqu'à 13 mL environ.

7. Système selon l'une quelconque des revendications précédentes, dans lequel ledit trou traversant (11) a une surface interne qui s'étend autour de ladite canule (8) le long d'au moins environ 0,5 cm de l'extension longitudinale de la canule (8).

8. Système selon l'une quelconque des revendications précédentes, dans lequel ladite canule (8) est sensiblement droite ; ledit trou traversant (11) est sensiblement droit.

9. Kit de manipulation d'un échantillon traité (TS) ; le kit est conçu pour obtenir un système (1) selon l'une quelconque des revendications précédentes ; le kit comprend :
un dispositif magnétique (7) ayant une chambre de traitement (2) et configuré pour créer un champ magnétique dans la chambre de traitement (2) ;
un récipient (3), conçu pour contenir ledit échantillon traité (TS) et pour être logé à l'intérieur de ladite chambre de traitement (2) et ayant au moins une paroi latérale (4), un fond (5) et une ouverture (6) à l'opposé du fond (5) ;
une canule (8), qui a une extrémité ouverte (9) ;
un dispositif d'alignement (10), qui est conçu pour être monté de manière sensiblement fixe dans la zone dudit récipient (3) et a un trou traversant (11) qui est conçu pour être mis en prise par ladite canule (8) et pour maintenir la canule (8) dans une position sensiblement fixe et avec une orientation sensiblement fixe et de telle sorte que la canule (8) est espacée de ladite paroi latérale (4) du récipient (3) ; et
un dispositif d'aspiration (12), conçu pour créer une dépression à l'intérieur de ladite canule (8) afin qu'une partie de l'échantillon traité (TS) soit transportée, à travers ladite canule (8), hors dudit récipient (3).

10. Kit selon la revendication 9 et comprenant un conduit (14), qui est conçu pour relier ladite canule (8) et ledit dispositif d'aspiration (12) de manière à transporter le fluide ; ledit dispositif d'alignement (10) est conçu pour être placé dans la zone de ladite ouverture (6) du récipient (3) ; en particulier, le conduit (14) a une première extrémité, qui est conçue pour être accouplée à la canule (8), et une seconde extrémité, qui est conçue pour être accouplée au dispositif d'aspiration (12) ; en particulier, le conduit (14) est souple.

11. Kit selon la revendication 9 ou 10, dans lequel ladite canule (8) a un diamètre interne allant jusqu'à 12 mm environ, en particulier jusqu'à 1 cm environ ; en particulier, la canule (8) a un diamètre interne d'au moins 0,3 mm environ, plus particulièrement, d'au moins 0,5 mm environ.

12. Kit selon l'une quelconque des revendications 9 à 11 et comprenant des particules ferromagnétiques (FP), qui sont pourvues d'au moins un anticorps conçu pour se lier aux particules (CE) du premier type ;
le kit comprend en outre au moins un marqueur choisi dans le groupe constitué : d'un marqueur pour les cellules tumorales, en particulier les CTC, d'un marqueur pour les globules blancs (WBC), d'un marqueur pour les noyaux des cellules, et d'une combinaison de ceux-ci ; en particulier, le marqueur est choisi dans le groupe constitué : de cytokératine, de CD45 (PTPRC - protéine tyrosine phosphatase, récepteur de type C), de DAPI (4',6-diamidino-2-phénylindole) et d'une combinaison de ceux-ci.

13. Procédé de manipulation d'un échantillon traité (TS) ; le procédé comprend :
une étape préliminaire, au cours de laquelle l'échantillon traité (TS), qui comprend des particules traitées (TP) comprenant chacune une particule (CE) d'un premier type et au moins une particule ferromagnétique (FP) liée à ladite particule (CE) du premier type, se trouve dans un récipient (3) ayant au moins une paroi latérale (4), un fond (5) et une ouverture (6) à l'opposé du fond (5) ;
une étape d'insertion, au cours de laquelle ledit récipient (3) est inséré dans une chambre de traitement (2) d'un dispositif magnétique (7) ;
une étape d'accouplement, qui fait suite à l'étape préliminaire et au cours de laquelle un dispositif d'alignement (10) ayant un trou traversant (11) est accouplé de manière stable audit récipient (3) ;
une étape de positionnement, qui fait suite à l'étape d'accouplement et au cours de laquelle une canule (8) ayant une extrémité ouverte (9) est amenée à passer à travers le trou traversant (11) sans toucher ladite paroi latérale (4) jusqu'à ce qu'elle atteigne une position donnée, dans laquelle ladite extrémité ouverte (9) est placée à l'intérieur dudit récipient (3) séparément de ladite paroi latérale (4) du récipient (3) ;
une étape d'activation, qui fait suite à l'étape d'insertion et au cours de laquelle le dispositif magnétique (7) crée un champ magnétique qui amène au moins une partie desdites particules traitées (TP) à entrer en contact avec ladite paroi latérale (4) ; et
une étape d'aspiration, qui fait suite à l'étape de positionnement et à l'étape d'activation, au cours de laquelle un dispositif d'aspiration (12) crée une dépression à l'intérieur de ladite canule (8) de sorte qu'une partie de l'échantillon traité (TS) est transportée, à travers ladite canule (8), hors dudit récipient (3) en laissant au moins une partie des particules traitées (TP) à l'intérieur du récipient.

14. Procédé selon la revendication 13 et mise en oeuvre au moyen du kit selon l'une quelconque des revendications 9 à 12 ; en particulier, l'échantillon traité (TS) comprend des particules d'un second type, dont au moins une grande partie est transportée, à travers ladite canule (8), hors dudit récipient (3) au cours de ladite étape d'aspiration ; en particulier, les particules (CE) du premier type sont des cellules tumorales circulantes.

15. Procédé selon la revendication 13 ou 14, dans lequel les particules (CE) du premier type sont choisies dans le groupe constitué : de CTC, de CEC (cellules endothéliales circulantes), de CMC (cellules de mélanome circulantes), de CMMC (cellules de myélome multiple circulantes), de tdEV (vésicules extracellulaires dérivées de tumeurs), d'exosomes, de cellules foetales, de cellules souches, d'autres cellules rares, de virus, de bactéries, de FFPE (d'éléments fixés au formol et imprégnés à la paraffine), de spermatozoïdes, de cellules sanguines, de cellules épithéliales, d'ADN, d'ARN, de microsphères.

16. Procédé selon l'une quelconque des revendications 13 à 15, dans lequel l'échantillon traité (TS) comprend des particules d'un autre type différent des particules (CE) du premier type ; le procédé comprend une étape de marquage, au cours de laquelle un marqueur est lié de manière sélective, notamment par rapport aux particules de l'autre type, aux particules (CE) du premier type ou de manière sélective, notamment par rapport aux particules (CE) du premier type, aux particules de l'autre type ; par exemple, le marqueur comprend des molécules fluorescentes avec une ou plusieurs longueurs d'onde données.

17. Processus de manipulation d'un échantillon initial comprenant des particules (CE) d'un premier type ; le processus comprend une étape de traitement, au cours de laquelle des particules ferromagnétiques (FP) susceptibles d'être sélectivement liées auxdites particules (CE) du premier type sont mélangées aux particules (CE) du premier type de manière à obtenir un échantillon traité (TS) comprenant des particules traitées (TP), chacune comprenant une particule (CE) d'un premier type et au moins une particule ferromagnétique (FP) liée à ladite particule (CE) du premier type ; et un procédé selon l'une quelconque des revendications 13 à 16.

18. Processus selon la revendication 17, dans lequel l'échantillon initial comprend un liquide biologique choisi dans le groupe constitué : de sang, de plasma, de salive, d'urine, d'une biopsie liquide, d'un écouvillon contenant des cellules en suspension, d'un milieu de culture contenant des cellules en suspension, et d'une combinaison de ceux-ci ; en particulier, le processus comprend également une étape d'élimination, qui précède l'étape de traitement et au cours de laquelle au moins une grande partie de l'échantillon initial est éliminée et séparée des particules (CE) du premier type.
